# EUROPÄISCHE PATENTSCHRIFT

(11) **EP 4 199 800 B1**
(45) Veröffentlichungstag und Bekanntmachung des Hinweises auf die Patenterteilung: **12.06.2024**
(21) Anmeldenummer: 22702644.0
(22) Anmeldetag: 20.01.2022
(51) Int. Cl.: A61B 1/012, A61B 1/00, A61B 1/018

(54) **ADAPTIERBARE ARBEITSKANALVORRICHTUNG, GEWEBECLIP-APPLIKATIONSADAPTER UND CHIRURGISCHES AUSRÜSTSYSTEM**
ADAPTABLE WORKING-CHANNEL APPARATUS, TISSUE-CLIP APPLICATION ADAPTER, AND SURGICAL EQUIPPING SYSTEM
APPAREIL À CANAL DE TRAVAIL ADAPTABLE, ADAPTATEUR D'APPLICATION DE PINCE À TISSU ET SYSTÈME D'ÉQUIPEMENT CHIRURGICAL

(30) Priorität: 21.01.2021 DE 102021101273
(43) Veröffentlichungstag der Anmeldung: 28.06.2023
(73) Patentinhaber: Ovesco Endoscopy AG, 72076 Tübingen (DE)
(72) Erfinder: SEIBT, Michael, 72070 Tübingen (DE); CAPUTO, Antonio, 72135 Dettenhausen (DE); HOFMANN, Nico, 73249 Wernau (DE); HO, Chi-Nghia, 72762 Reutlingen (DE)
(74) Vertreter: Winter, Brandl - Partnerschaft mbB
(86) Internationale Anmeldenummer: PCT/EP2022/051233
(87) Internationale Veröffentlichungsnummer: WO 2022/157244

(56) Entgegenhaltungen:
- WO-A1-2008/098124
- DE-A1-102017 107 546
- DE-A1-102017 112 896
- US-A1- 2013 310 641
- US-A1- 2019 183 471

## Beschreibung

Die vorliegende Offenbarung betrifft eine adaptierbare Arbeitskanalvorrichtung zur adaptiven Montage an einem Endoskop mit zumindest einem externen Arbeitskanal, der an seinem proximalen Endabschnitt an eine erste Montagekupplung angeschlossen ist, einer separat zum Arbeitskanal ausgebildeten Griff-Montagemanschette, welche dafür vorgesehen ist, einen Endoskopschaft oder einen Endoskopgriff manschettenartig zu umgreifen und welche an ihrer radial äußeren Umfangsseite zumindest eine zweite Montagekupplung hat, die ausgebildet ist, um mit der ersten Montagekupplung in einen lösbaren Montageeingriff zu kommen. Ferner betrifft die vorliegende Offenbarung einen distalen Gewebeclip-Applikationsadapter sowie ein chirurgisches Ausrüstsystem für ein Endoskop mit der adaptierbaren Arbeitskanalvorrichtung und dem Gewebeclip-Applikationsadapter.

### Stand der Technik

Medizinische Endoskope werden kommen bei vielen unterschiedlichen Patienten-Eingriffen zum Einsatz und unterliegen entsprechend vielfältigen Anforderungen. Um der großen Anzahl verschiedener, hochspezialisierter Eingriffe gerecht zu werden, gibt es hochspezialisierte Endoskope und/oder Adaptersysteme für zusätzliche Arbeitskanäle, welche ein Bereitstellen zusätzlicher Instrumente und/oder Funktionalitäten ermöglichen. Derartige Adaptersysteme sind zum Ausrüsten insbesondere von Standard-Endoskopen konfiguriert. Sie müssen schnell und leicht an dem Endoskop angebracht werden können und sicher daran befestigt sein. Bevorzugt sind sie kostengünstig als Einwegteile herstellbar und somit einfach aufgebaut.

Ein Beispiel für eine zusätzliche Schlauchanordnung, die über einen proximalen Konnektor mit einem Endoskopgriff koppelbar ist, findet sich in DE 10 2009 014 178 A1. Diese Druckschrift offenbart eine Klemmhülse, die an einem Endoskopgriff aufspannbar ist und einen Klammerspalt mit einer Noppe aufweist, sowie einen externen Arbeitskanal, der zur Montage am Klammerspalt Flügel mit einer Eingriffsmulde zum Eingriff mit der Noppe aufweist. Diese Verbindung ist jedoch entweder schwergängig und somit nur schwer und ggf. ruckartig zu bedienen oder aber leichtgängig und instabil. Da beim Verbinden ein Gegendruck aufgebaut werden muss, sind ferner zwei Hände notwendig, um den externen Arbeitskanal an dem Endoskopgriff zu befestigen. Zudem kann der Drehring ausleiern und weitere Instabilitäten hervorrufen. Bei vielen Patientenbehandlungen oder Operationen ist jedoch sowohl eine hohe Stabilität der Verbindung als auch eine einfache und sichere Montage und Demontage erforderlich.

Weiterer Stand der Technik ist z.B. aus WO 2008/098124 A1 bekannt. Darin ist eine Vorrichtung zur Befestigung eines ersten medizinischen Geräts an einem zweiten medizinischen Gerät beschrieben, die es dem Arzt ermöglichen, nur ein einziges Gerät zu ergreifen, während das andere Gerät sicher an dem ergriffenen Gerät befestigt bleibt. Die Vorrichtungen haben über eine Basis, die sich leicht von dem Teil, der an der zweiten medizinischen Vorrichtung angebracht ist, koppeln und abkoppeln lässt, so dass die zweite medizinische Vorrichtung bei Bedarf von der ersten medizinischen Vorrichtung abgekoppelt werden kann, ohne die Vorrichtung vollständig von der zweiten medizinischen Vorrichtung zu entfernen. Die zweite medizinische Vorrichtung kann z.B. durch einen Greifer mit zwei federgelagerten Branchen gehalten werden.

Darüber hinaus werden Adaptersysteme, die aus dem Stand der Technik bekannt sind, den durch spezialisierte Eingriffe definierten Anforderungen häufig nicht gerecht. So ist beispielsweise aus der Druckschrift DE 10 2017 112 896 A1 ein Adaptersystem für eine distale Endoskopspitze bekannt, welches einen Aufsatz mit einem darauf getragenen und herunterschiebbaren Gewebeclip hat. Allerdings ist dieser Aufsatz in seinen Einsatzmöglichkeiten stark beschränkt. Ferner hat sich herausgestellt, dass dieses Adaptersystem beispielsweise beim Herstellen einer künstlichen Anastomose (d.h. beim Legen einer Verbindung zwischen zwei anatomischen Hohlräumen, wie z.B. zwischen Magen und Dünndarm) problematisch sein kann. Insbesondere kann es bei einer operativ hergestellten Anastomose vorkommen, dass eine Öffnung der Verbindung zu groß ist. Bei einer Magen-Dünndarm-Anastomose kann somit beispielsweise Essen innerhalb des Verdauungstrakts zu schnell und somit entsprechend unzureichend verdaut weitertransportiert werden. Andererseits kann die Öffnung zu klein sein. Dann kann es vorkommen, dass Nahrung zu langsam weitertransportiert wird. In beiden Fällen kann dies zu Verdauungsproblemen führen.

Ein weiteres Adaptersystem zum Applizieren einer Gewebebefestigungsvorrichtung ist z.B. aus US 2019/0183471 A1 bekannt.

### Zusammenfassung der Erfindung

Die der vorliegenden Offenbarung zugrunde liegende Aufgabe besteht darin, Nachteile des Stands der Technik zu verbessern oder zu beseitigen. Insbesondere soll ein adaptierbares Arbeitskanalsystem und/oder ein Gewebeclip-Applikationsadapter und/oder ein chirurgisches Ausrüstsystem für ein Endoskop bereitgestellt werden, das einfach, kostengünstig und sicher zu montieren und zu demontieren ist und/oder zusätzliche Funktionalitäten zuverlässig und einfach bereitstellen kann.

Die der Offenbarung zugrunde liegende Aufgabe wird durch eine adaptierbare Arbeitskanalvorrichtung mit den Merkmalen des Anspruchs 1 gelöst.

Genauer ausgedrückt, wird die der Erfindung zugrundeliegende Aufgabe gelöst durch eine adaptierbare Arbeitskanalvorrichtung zur adaptiven Montage an einem Endoskop. Die adaptierbare Arbeitskanalvorrichtung hat zumindest einen externen Arbeitskanal, der an seinem proximalen Endabschnitt an eine erste Montagekupplung angeschlossen ist. Ferner hat die adaptierbare Arbeitskanalvorrichtung eine separat zum Arbeitskanal ausgebildete Griff-Montagemanschette, welche dafür vorgesehen ist, einen Endoskopschaft oder einen Endoskopgriff manschettenartig zu umgreifen. An der radial äußeren Umfangsseite der Griff-Montagemanschette ist zumindest eine zweite Montagekupplung bereitgestellt, die dazu ausgebildet ist, um mit der ersten Montagekupplung in einen lösbaren Montageeingriff zu kommen. Die erste Montagekupplung hat zwei in einen Klemmeingriff federvorgespannte Klemmbranchen, die wäscheklammerartig aneinanderscharniert sind und dabei jeweils einen Klemm-Branchenabschnitt und einen manuellen Betätigungs-Branchenabschnitt bilden. Die zweite Montagekupplung hat eine Montageschiene, die sich in Längsrichtung der Griff-Montagemanschette erstreckt, und zumindest einen Hinterschnitt bildet. Der Hinterschnitt ist derart dimensioniert, dass er formschlüssig von den Klemmbranchen der ersten Montagekupplung umgreifbar ist. Die adaptierbare Arbeitskanalvorrichtung ermöglicht vorteilhafter Weise ein einfaches, ruckfreies sowie stabiles Koppeln eines externen Arbeitskanals an einen Endoskopgriff.

In anderen Worten ausgedrückt, wird eine adaptierbare Arbeitskanalvorrichtung mit einem externen Arbeitskanal bereitgestellt, welcher über einfaches Montagesystem an einem Endsokopgriff wahlweise befestigbar oder lösbar ist. Der (externe) Arbeitskanal und eine am Endoskopgriff befestigbare Griff-Montagemanschette haben entsprechend eine erste und eine zweite Montagekupplung, die zur lösbaren Kopplung miteinander ausgebildet sind. Die erste Montagevorrichtung am Arbeitskanal bildet eine Klammer mit zwei über ein Wippgelenk zueinander elastisch winkelverstellbaren (d.h., wäscheklammerartig verbundenen) Klemmbranchen. Die Klemmbranchen haben jeweils ein erstes freies Ende, das an einer Klemmseite über das Wippgelenk vorsteht, um einen Klemm-Branchenabschnitt mit zumindest einem ersten Eingriffselement, wie einem Fortsatz oder einer Öffnung, zu bilden. Ein zweites freies Ende der Klemmbranchen bildet einen Betätigungs-Branchenabschnitt, der zum Öffnen der gegenüberliegenden Klemm-Branchenabschnitte durch einen Anwender mittels einer manuellen Druckbetätigung konfiguriert ist, und der dem Klemm-Branchenabschnitt bezüglich des Wippgelenks gegenüberliegt. Die zweite Montagekupplung hat einen spangen- oder hülsenförmigen Abschnitt, welcher um den Endsokopgriff oder -schaft geschnappt/aufgesetzt werden kann. Der spangen- oder hülsenförmige Abschnitt hat Längsleisten/Montageschienen mit zumindest einem zweiten Eingriffselement in Form einer Öffnung oder eines Vorsprungs, das zum Eingriff mit einem kompatiblen ersten Eingriffselement der Klemmbranche(n) vorgesehen ist. Der Klemm-Branchenabschnitt und der Betätigungs-Branchenabschnitt der jeweiligen Klemmbranchen sind insbesondere steif miteinander verbunden.

Vorteilhafte Ausgestaltungen sind Gegenstand der Unteransprüche und werden nachfolgend genauer beschrieben.

Das Wippgelenk bzw. die Anscharnierung kann unmittelbar am Arbeitskanal bzw. an einer Anschlusshülse des Arbeitskanals bereitgestellt sein oder kann über einen Abstandhalter in radialer Richtung bezüglich des Arbeitskanals beabstandet sein. Durch einen solchen Abstandhalter kann gewährleistet sein, dass ein proximaler Zugang zu dem Arbeitskanal derart von dem Endoskop beabstandet ist, dass er leicht zugänglich ist. Ferner sind die Betätigungs-Branchenabschnitte bevorzugt derart angeordnet, dass diese zum Öffnen und/oder Schließen einhändig bedienbar sind.

Die Klemmbranchen der ersten Montagekupplung bilden vorzugsweise an ihren Klemm-Branchenabschnitten einander zugewandte Eingriffselemente, wie hakenförmige Fortsätze oder Öffnungen aus, welche zum Eingriff hinter den zumindest einen Hinterschnitt der zweiten Montagekupplung ausgebildet sind. Insbesondere können Kontaktflächen des Hinterschnitts und der Eingriffselemente senkrecht zu den Klemmbranchen verlaufend ausgebildet sein, sodass eine stabile Anlage zwischen den Montagekupplungen entsteht. Dies ist insbesondere möglich, da durch die wäscheklammerartige Anscharnierung die beiden Klemmbranchen eine ausladende Bewegung durchführen, sodass die jeweiligen Eingriffselemente eine große Querbewegung durchführen und somit die Eingriffselemente eine relativ große Erstreckung quer zur Kemmbranchenlängsrichtung haben können, um relativ große derartige Kontakt-/Anlageflächen bereitzustellen. Besonders bevorzugt ist es, wenn die Fortsätze entlang einer gesamten Breite der jeweiligen Klemm-Branchenabschnitte ausgebildet sind, um eine möglichst lange und somit stabile Eingriffskante bereitzustellen.

Des Weiteren hat es sich als zweckmäßig erwiesen, wenn die freien Enden der Klemm-Branchenabschnitte stirnseitig an einander zugewandten Kanten angeschrägt sind, um zwischen sich einen Einführkeil auszubilden.

Vorzugsweise sind zumindest zwei Hinterschnitte bereitgestellt, welche einander gegenüberliegend auf beiden in Umfangsrichtung gewandten Seitenflanken der Montageschiene angeordnet sind. Der/Die Hinterschnitt/e ist/sind jeweils bevorzugt als eine Durchgangsöffnung ausgebildet, welche sich tangential zur Längsrichtung der Griffmontagemanschette durch die Griff-Montagemanschette hindurch erstreckt. Dies ist einfach zu fertigen und stellt eine besonders stabile Koppelstruktur auf. Die Griff-Montagemanschette kann bevorzugt eine im Wesentlichen zylindrische oder kegelabschnittsartige Form aufweisen, welche nach radial außen ausgewölbt ist, um die Montageschiene zu bilden. Auf diese Weise wird eine einfache Struktur erzielt und die Griff-Montagemanschette ist einfach und kostengünstig zu fertigen.

Weiter bevorzugt erstrecken sich die Klemm-Branchenabschnitte ausgehend von der Anscharnierung bzw. dem Wippgelenk schräg voneinander weg. In anderen Worten ausgedrückt, schließen sie zwischen sich einen spitzen Winkel ein. Demgemäß können die Klemm-Branchenabschnitte auch um eine Montageschiene greifen, die besonders breit und stabil ausgebildet ist. An den Betätigungs-Branchenabschnitte können außenseitig (d.h., voneinander abgewandt) Anti-Rutsch-Strukturen, wie Rillen oder Stege, ausgebildet sein, um einen sichereren Halt bei der manuellen Betätigung der Betätigungs-Branchenabschnitte bereitzustellen.

Es hat sich als zweckmäßig erwiesen, wenn die erste Montagekupplung eine Anschlusshülse ausbildet, an deren distalem Ende der Arbeitskanal angeschlossen ist. Bevorzugt ist ferner an einem proximalen Ende der Anschlusshülse ein Luer-Adapter oder Luer-Lock-Adapter angeschlossen. Der Luer-(Lock-) Adapter kann mit einem Biopsieventil ausgestattet sein. Alternativ kann die Anschlusshülse ein integrales Biopsieventil ausbilden. Dies ermöglicht es beispielsweise, den externen Arbeitskanal beim Biopsieren oder Spülen abzudichten und erweitert die möglichen Funktionalitäten der vorliegenden adaptierbaren Arbeitskanalvorrichtung. Die Anschlusshülse bildet insbesondere einen Längsdurchgang/-kanal aus, welcher den Arbeitskanal mit einer proximalen Eingangsöffnung der Anschlusshülse verbindet.

Bevorzugt sind die Klemmbranchen jeweils an einer Position zwischen dem Klemm-Branchenabschnitt und dem Betätigungs-Branchenabschnitt der entsprechenden Klemmbranche über einen Verbindungssteg elastisch miteinander verbunden, der schräg oder senkrecht zu den Klemmbranchen verläuft. Ein solcher Verbindungssteg ermöglicht zum einen eine einfache und einstückige, montagefreie Ausgestaltung der Anscharnierung bzw. des Wippgelenks, die/das die beiden Klemmbranchen miteinander verbindet. Ferner kann der Verbindungssteg die beiden Klemmbranchen zueinander beabstanden. Dies ermöglicht einen größeren Betätigungsweg beim Öffnen und Schließen der Klemmbranchen über eine Betätigung der Betätigungs-Branchenabschnitte.

Weiter bevorzugt ist der Verbindungssteg mit der Anschlusshülse z.B. über einen zentralen Balken als einem Abstandhalter verbunden, welcher zentral an dem Verbindungssteg ansetzt. Eine Länge des Abstandhalters ist vorzugsweise derart bemessen, dass er den Verbindungssteg von der Anschlusshülse beabstandet, um die Betätigungsbrachenabschnitte ungehindert durch die Anschlusshülse frei betätigen zu können. Ferner ist eine Länge des Abstandhalters derart bemessen, dass ein freies Ende der Betätigungs-Branchenabschnitte nahe an der Anschlusshülse liegt, derart, dass ein Anwender mit einer Hand um die Anschlusshülse an die Betätigungs-Branchenabschnitte greifen kann. Der Abstandhalter kann für die Betätigungsbrachenabschnitte als Endanschlag dienen, um eine übermäßige Betätigung und eine dadurch bedingte Beschädigung der ersten Montagekupplung zu vermeiden.

Zweckmäßigerweise können die Klemmbranchen und die Anschlusshülse derart zueinander ausgerichtet sein, dass eine Längsachse der Anschlusshülse und eine Längsachse der Griff-Adaptermanschette in einem Zustand, in welchem die erste und die zweite Montagekupplung gekoppelt sind, schräg zueinander ausgerichtet sind. Vorzugsweise schließen diese Längsachsen zwischen sich einen Winkel von vorzugsweise 10° bis 50°, weiter vorzugsweise 20° bis 25° ein. Somit ist ein proximaler Arbeitskanalzugang von dem Endoskop abgewandt ausgerichtet. Der proximale Arbeitskanalzugang ist somit leichter zugänglich für einen Nutzer. Insbesondere ist dies vorteilhaft in Kombination mit dem vorstehend beschriebenen Abstandhalter, da die schräge Anordnung der Anschlusshülse es ermöglicht, dass der Arbeitskanal von einem maximalen Abstand diagonal in Richtung des Endoskopschafts führbar ist. Der Arbeitskanal ist insbesondere an dem Endoskopschaft außenseitig befestigt und in Richtung distal geführt.

Nach einem vorteilhaften Aspekt ist der Verbindungssteg derart breiter als die Klemmbranchen, dass er seitlich (d.h. in proximaler und/oder distaler Richtung bzw. in einer Längsrichtung bezüglich des Arbeitskanals) über die Klemmbranchen vorsteht. Besonders von Vorteil ist es, wenn ferner die Griff-Montagemanschette zumindest zwei quer zu ihrer Längsrichtung verlaufende und in der Längsrichtung beabstandete Stützkanten ausbildet, deren Abstand zueinander im Wesentlichen einer Breite des Verbindungsstegs entspricht. Zwischen den Stützkanten ist bevorzugt der Hinterschnitt ausgebildet. Auf diese Wiese können Kräfte, welche über die erste und zweite Montagekupplungen übertragen werden, auf unterschiedliche Kupplungselemente verteilt werden. Beispielsweise können Kräfte, welche in Längsrichtung des Arbeitskanals bzw. der Montagehülse wirken, über die Stützkanten und seitliche Kanten der des Verbindungsstegs übertragen werden, und belasten nicht die Klemm-Branchenabschnitte. Die Klemm-Branchenabschnitte übertragen in diesem Fall lediglich Kräfte, welche in radialer Richtung zwischen den ersten und zweiten Montageabschnitten wirken.

Bevorzugt bildet die Montageschiene der Griff-Montagemanschette mehrere Hinterschnitte aus, die in der Längsrichtung zueinander versetzt angeordnet sind. Dies ermöglicht es, Arbeitskanäle unterschiedlicher Länge bereitzustellen, welche jeweils mit der gleichen Griff-Montagemanschette kombinierbar sind. Genauer ausgedrückt, kann beispielsweise ein relativ kurzer Arbeitskanal über einen weiter distal angeordneten Hinterschnitt an der Griff-Adaptermanschette befestigt werden und/oder kann ein relativ langer Arbeitskanal über einen weiter proximal gelegenen Hinterschnitt befestigt werden. Auf diese Weise ist die Kombination der adaptierbaren Arbeitskanalvorrichtung mit Endoskopen verschiedener Länge vereinfacht.

Vorteilhafter Weise bildet die Griff-Montagemanschette mehrere Montageschienen aus, die in einer Umfangsrichtung der Griff-Montagemanschette zueinander versetzt, vorzugsweise einander diametral gegenüberliegend, angeordnet sind. Dies ermöglicht eine flexiblere Anordnung des Arbeitskanals am Endoskop. Ferner kann die adaptierbare Arbeitskanalvorrichtung somit mehrere Arbeitskanäle mit entsprechenden ersten Montagekupplungen aufweisen.

Nach einem weiteren bevorzugten Aspekt hat die Griff-Montagemanschette eine Aufnahmeöffnung, welche dazu ausgebildet ist, einen Radialfortsatz des Endoskopgriffs aufzunehmen. Der Radialfortsatz ist beispielsweise ein Ausgang eines Endoskopeigenen, internen Arbeitskanals am Endoskopgriff. Somit ist die Griff-Montagemanschette formschlüssig an dem Endoskopgriff zumindest in Längsrichtung und/oder in Umfangsrichtung der Griff-Adaptermanschette lagefestgelegt. Dies ermöglicht eine besonders einfache und stabile, ausleierbeständige Verbindung zwischen der Griff-Montagemanschette und dem Endsokopgriff.

Ferner ist es von Vorteil, wenn die Griff-Montagemanschette einen durchgehenden Längsspalt aufweist und zumindest teilweise elastisch ausgebildet ist, derart, dass die Griff-Montagemanschette spangenartig auf den Endoskopschaft/-griff aufschnappbar ist. Dadurch wird ermöglicht, dass die Griff-Montagemanschette eine Spannkraft auf den Endsokopschaft/-griff aufbringen kann. Insbesondere kann ein dem Längsspalt gegenüberliegender Umfangsbereich der Griff-Montagemanschette einen oder mehrere Durchbrüche aufweisen, um einen zumindest teilweise elastischen Bereich zu bilden. In anderen Worten ausgedrückt, kann eine Manschettenstruktur bereichsweise geschwächt sein, um eine höhere Elastizität als andere Bereiche der Griff-Montagemanschette zu haben. Besonders vorteilhaft ist es, wenn der Längsspalt an einer der Aufnahmeöffnung diametral gegenüberliegenden Seite ausgebildet ist, und durch die Aufnahmeöffnung die geschwächte Manschettenstruktur bereitgestellt ist.

Nach einem weiteren vorteilhaften Aspekt weist die Griff-Montagemanschette eine Bandhalterung auf, an welcher ein erstes Ende eines Verschlussbands angebracht oder anbringbar ist. Ferner kann die Griff-Montagemanschette einen Bandbefestigungsabschnitt aufweisen, welcher dazu ausgebildet ist, einen Verschlussabschnitt des Verschlussbands in einer Position zu halten, in welcher es derart um die Griff-Montagemanschette gelegt ist, dass es sich über den Längsspalt erstreckt. In anderen Worten ausgedrückt, ist ein Verschlussband bereitgestellt, welches derart an der Griff-Montagemanschette angeordnet ist, dass es den Längsspalt und zumindest einen Teil der Griff-Montagemanschette umspannt, um eine Spannung auf einen innerhalb der Griff-Montagemanschette angeordneten Edoskopabschnitt aufzubringen. Bevorzugt ist die Bandhalterung und/oder der Bandbefestigungsabschnitt radial außen an einer oder entsprechend an zwei einander gegenüberliegenden Montageschienen angebracht. Somit wird ein Hebel bereitgestellt, welcher es ermöglicht, eine höhere Spannkraft des Verschlussbands um die Griff-Montagemanschette aufzubringen. Der Verschlussabschnitt kann beispielsweise als ein Ratschenband, nach einem gelochten Gürtelprinzip oder aus Klettband ausgebildet sein, wobei der Bandbefestigungsabschnitt entsprechend anzupassen ist.

Alternativ oder zusätzlich wird die der Offenbarung zugrunde liegende Aufgabe durch einen Gewebeclip-Applikationsadapter nach Anspruch 10 gelöst.

Genauer ausgedrückt, ist ein Gewebeclip-Applikationsadapter bereitgestellt, der nach Art eines Kappenaufsatzes für ein medizinisches Endoskop, d.h. separat dazu, ausgebildet ist. Der Gewebeclip-Applikationsadapter hat einen hülsenartigen Grundkörper. Dieser weist einen proximalen Endoskop-Halteabschnitt auf, der dazu ausgebildet ist, an einem distalen Kopf des medizinischen Endoskops angebracht zu werden. Ferner weist der Grundkörper einen Gewebeclip-Halteabschnitt auf, der dazu ausgebildet ist, auf seiner radial äußeren Umfangsfläche einen Gewebeclip derart zu tragen, dass dieser in distaler Richtung von dem Gewebeclip-Halteabschnitt entfernbar ist. Insbesondere ist der Grundkörper in distaler Richtung offen, um zu ermöglichen, dass Instrumente oder Ähnliches durch den Grundkörper zu einer Behandlungsstelle / in einen Hohlraum eines Patienten vorgeschoben werden kann und/oder dass Patientengewebe in den Grundkörper eingeholt / hineingezogen werden kann.

Der Gewebeclip-Applikationsadapter hat des Weiteren einem ersten Adapter-eigenen Arbeitskanal-Anschluss mit einem ersten Innenkanal. Der erste Innenkanal erstreckt sich derart durch den Grundkörper, dass dessen proximales erstes Kanalende sich an einer Außenseite des Grundkörpers öffnet und dass dessen distales erstes Kanalende in einem Innenraum innerhalb des Grundkörpers mündet. In anderen Worten ausgedrückt erstreckt sich ein Durchgang, an welchen ein separater Arbeitskanal angeschlossen ist oder anschließbar ist, aus einer proximalen Richtung kommend von radial außerhalb des Grundkörpers diagonal, d.h. in Richtung distal und radial innen, durchgängig durch eine Wand des Grundkörpers. Optional kann eine innere Auskragung innerhalb des zweiten Innenkanals vorgesehen sein, um einen anzuschließenden bzw. angeschlossenen externen Arbeitskanal in distaler Richtung lagefestzulegen.

Dies hat den Vorteil, dass ein zusätzlicher Arbeitskanal bereitgestellt werden kann, durch welchen Instrumente, Spülschläuche oder Ähnliches bis zu einem Raum unmittelbar vor dem eigentlichen Endoskop vorgeschoben werden können. Insbesondere sind eingeschobene Instrumente etc. durch den diagonalen Verlauf des ersten Innenkanals bereits in eine entsprechend radiale Richtung ausgerichtet, was beispielsweise für Spülprozesse oder zum Einziehen von Gewebe in den Grundkörper hinein von Vorteil sein kann.

Insbesondere ist dies von Vorteil für ein definiertes, gezieltes Setzen eines Gewebeclips. Hierfür kann ein Greifinstrument durch den entsprechenden externen Arbeitskanal und den ersten Innenkanal in den Grundkörper vorgeschoben werden und distal aus dem Grundkörper austreten. Dort kann es in einem Bereich, der unmittelbar im Sichtfeld einer Optik des Endoskops liegt, gezielt Patientengewebe greifen und dieses ins Innere des Grundkörpers einziehen. Wird anschließend der Gewebeclip in Richtung distal von dem Gewebeclip-Halteabschnitt entfernt / heruntergeschoben, erfolgt eine gezielte, definierte Klemmung eines bestimmten Abschnitts von Patientengewebe. Das Greifinstrument kann ein mechanisches, z.B. zangenartiges, Instrument oder ein Sauginstrument sein, dass das Patientengewebe durch einen Ansaugdruck festhalten/greifen kann.

Ein zweiter Adapter-eigener Arbeitskanal-Anschluss des Gewebeclip-Applikationsadapters hat einen zweiten Innenkanal, der sich derart entlang des Grundkörpers erstreckt, dass ein proximales zweites Kanalende und ein distales zweites Kanalende des zweiten Innenkanals sich an einer Außenseite des Grundkörpers öffnen. Ferner ist das distale zweite Kanalende proximal zu dem Gewebeclip-Halteabschnitt angeordnet. In anderen Worten ausgedrückt ist ein (Innen-) Kanal bereitgestellt, der sich im Wesentlichen linear entlang einer Außenumfangsfläche des hülsenartigen Grundkörpers erstreckt, um (unabhängig von dem ersten Innenkanal) einen weiteren externen Arbeitskanal anzuschließen, der aus proximaler Richtung kommend an den Gewebeclip-Applikationsadapter angeschlossen ist oder anschließbar ist. Optional kann eine Auskragung innerhalb des zweiten Innenkanals vorgesehen sein, um einen anzuschließenden bzw. angeschlossenen externen Arbeitskanal in distaler Richtung lagefestzulegen.

Somit ermöglicht es der vorliegende Gewebeclip-Applikationsadapter, Instrumente, Spülschläuche, spezielle Optiken etc. bereitstellen zu können, die eine Umgebung des zu klammernden Patientengewebes beobachten oder manipulieren können, ohne dass die Funktionalität des Gewebeclip-Applikationsadapters zum Klammern von Patientengewebe mittels des Gewebeclips eingeschränkt wird oder das adaptierte Endoskop deutlich eingeschränkt wäre. Dies ist insbesondere beim Durchführen einer Anastomose von Vorteil, bei welcher eine Gewebeöffnung definierter Größe neben dem durch den Gewebeclip zu klammerden Gewebe offen bleiben soll.

In diesem Anwendungsbeispiel einer Anastomosesetzung kann beispielsweise ein Spreizinstrument, wie z.B. ein Ballonkatheter, durch den entsprechend angeschlossenen externen Arbeitskanal und den zweiten Innenkanal bis in die vorzusehende Gewebeöffnung vorgeschoben werden. Dort kann das Spreizinstrument auf eine vorbestimmte Größe / einen vorbestimmten Durchmesser aufgespreizt werden. Wird anschließend zu klammerndes Patientengewebe unmittelbar neben dem Spreizinstrument, wie z.B. vorstehend beschrieben, durch ein Greifinstrument gegriffen und in den Grundkörper gezogen, spannt sich das Patientengewebe um das Spreizinstrument. Wenn anschließend die Klammer gesetzt ist und das Spreizinstrument entfernt wird, verbleibt neben der geklapperten Gewebestelle eine Gewebeöffnung, welche im Wesentlichen der vorbestimmten Größe / dem vorbestimmten Durchmesser des Spreizinstruments im gespreizten Zustand entspricht.

Bevorzugt ist unmittelbar proximal zu dem Gewebeclip-Halteabschnitt eine Schiebehülse als eine Gewebeclip-Abschiebevorrichtung in Längsrichtung verschiebbar auf dem Grundkörper gelagert, um den Gewebeclip in distaler Richtung von dem Gewebeclip-Halteabschnitt herunterzuschieben. In anderen Worten ausgedrückt, ist eine Schiebehülse zum Abschieben des Gewebeclips zwischen dem Gewebeclip-Halteabschnitt und dem distalen Ende des zweiten Innenkanals bereitgestellt. Dies ermöglicht ein besonders einfaches, gleichmäßiges und effektives Abschieben des Gewebeclips. Da die Schiebehülse ferner nur wenig radialen Bauraum benötigt, können sich z.B. die Gewebeclip-Abschiebevorrichtung und die Spreizvorrichtung einander nicht in den Weg kommen.

Nach einem vorteilhaften Aspekt hat eine distale Stirnkante des Grundkörpers (d.h. ein am weitesten distal angeordnete Ende des Gewebeclip-Applikationsadapters) zwei Rücksprünge, welche einander (vorzugsweise diametral) gegenüberliegen und welche sich in proximaler Richtung erstrecken oder ausgespart sind. In anderen Worten ausgedrückt, ist die Stirnkante maulartig ausgebildet, wobei die Rücksprünge Mundwinkel der maulartigen Form bilden. Dies ist insbesondere von Vorteil um das Patientengewebe besonders definiert in den Grundkörper ziehen und dabei in die Rücksprünge falten zu können. Dies ermöglicht eine besonders definierte, längsgerichtete Klammerung des Patientengewebes.

Besonders bevorzugt sind die Rücksprünge jeweils in einander entgegengesetzten Umfangsrichtungen des Grundkörpers versetzt zu zweiten Adapter-eigenen Arbeitskanal-Anschluss angeordnet, vorzugsweise um im 70° bis 110°, weiter vorzugsweise um 90° dazu versetzt. Das heißt, einer der Rücksprünge ist in einer ersten Umfangsrichtung um einen entsprechenden Winkel zu dem zweiten Arbeitskanal-Anschluss versetzt und der jeweils andere Rücksprung ist um einen entsprechenden Winkel in der entgegengesetzten Umfangsrichtung zu dem zweiten Arbeitskanal-Anschluss versetzt. Somit liegen sich die beiden Rücksprünge im Wesentlichen diametral gegenüber und der zweite Arbeitskanal-Anschluss ist an einer Umfangsposition zwischen den beiden Rücksprüngen angeordnet. In anderen Worten ausgedrückt, sind der zweite Arbeitskanal-Anschluss und die Rücksprünge derart zueinander ausgerichtet, dass beim Operieren einer Anastomose das zu klammernde Patientengewebe im Wesentlichen tangential zu einer vorzusehenden Gewebeöffnung gefaltet wird. Eine anschließende Klammerung des Gewebes erfolgt somit besonders genau und zielgerichtet.

Ferner ist es von Vorteil, wenn der Gewebeclip-Halteabschnitt zwei einander diametral gegenüberliegende Spreizstützflächen ausbildet, welche dazu konfiguriert sind, den Gewebeclip in einer vorbestimmten Lage aufgespreizt zu halten, wobei eine der Spreizstützflächen zu dem zweiten Adapter-eigenen Arbeitskanal-Anschluss fluchtend bzw. an einer gleichen Umfangsposition des Grundkörpers angeordnet ist. In anderen Worten ausgedrückt, ist ein Gewebeclip, für welchen der Gewebeclip-Halteabschnitt angepasst ist, bärenfallenartig ausgebildet und hat zwei kieferartige Krallenabschnitte, die in Richtung distal ausgerichtet sind. Einer der Krallenabschnitte ist an der gleichen Umfangsposition wie der zweite Arbeitskanal-Anschluss angeordnet. Dies ist von Vorteil, da somit beim Operieren einer Anastomose einer der Krallenabschnitte, der dazu ausgebildet ist, das zu klammernde Patientengewebe fest zu greifen und festzukrallen, unmittelbar neben der vorzusehenden Gewebeöffnung in das Patientengewebe greift. Auf diese Weise kann eine Menge von "losem" Gewebe, das zwischen der Gewebeöffnung und der Klammerung überbleibt und zu Ungenauigkeiten bei der Einstellung einer Größe der Gewebeöffnung führen kann, minimiert werden und Verdauungsbeschwerden des Patienten vermieden werden.

Alternativ oder zusätzlich wird die der Offenbarung zugrunde liegende Aufgabe durch ein chirurgisches Ausrüstsystem nach Anspruch 15 gelöst.

Genauer ausgedrückt, wird ein chirurgisches Ausrüstsystem für ein Endoskop mit einer vorstehend beschriebenen adaptierbaren Arbeitskanalvorrichtung und einem vorstehend beschriebenen Gewebeclip-Applikationsadapter bereitgestellt. Der erste Adapter-eigene Arbeitskanal-Anschluss ist mit einem distalen Endabschnitt des externen Arbeitskanals der adaptierbaren Arbeitskanalvorrichtung als einem ersten externen Arbeitkanal gekoppelt. Des Weiteren weist das Ausrüstsystem ein Greifinstrument auf, welches durch den ersten externen Arbeitskanal in Richtung distal eingeschoben ist oder dazu ausgebildet ist, in den ersten externen Arbeitskanal in Richtung distal eingeschoben zu werden. Somit ist das Greifinstrument durch den Innenraum des Grundkörpers des Gewebeclip-Applikationsadapters vorschiebbar.

Darüber hinaus ist ein zweiter externer Arbeitskanal bereitgestellt, welcher an seinem proximalen Endabschnitt an eine dritte Montagekupplung zur Montage an der Griff-Montagemanschette der adaptierbaren Arbeitskanalvorrichtung angeschlossen ist. Ein distaler Endabschnitt des zweiten Arbeitskanals ist mit dem zweiten Adapter-eigenen Arbeitskanal-Anschluss des Gewebeclip-Applikationsadapters gekoppelt. Ferner weist das Ausrüstsystem ein Spreizinstrument auf, insbesondere einen Ballonkatheter, welches in den zweiten externen Arbeitskanal in Richtung distal eingeschoben ist oder dazu ausgebildet ist, in den zweiten externen Arbeitskanal eingeschoben zu werden. Somit ist das Spreizinstrument radial außerhalb des Grundkörpers des Gewebeapplikations-Adapters bereitstellbar. Durch dieses Ausrüstsystem kann vorteilhafterweise eine optimale Einstellung einer verbleibenden Gewebeöffnung bei einer Anastomose erreicht werden, wie vorstehend bereits genauer beschrieben wurde. Bevorzugt sind der erste und der zweite Arbeitskanal sowie die jeweils zugeordneten ersten Montagekupplungen unterschiedlich markiert, beispielsweise unterschiedlich farbig, um die Arbeitskanäle unterscheiden zu können.

Zusammenfassend wird die der Erfindung zugrunde liegende Aufgabe wie folgt gelöst. Der Handgriff (Griff-Montagemanschette) kann mehrere Funktionen haben. Zum einen ist dieser bevorzugt wie eine Spange konstruiert, die einfach auf das Endoskop (insbesondere Endoskopgriff) aufgeschoben bzw. davon abgezogen werden kann. Durch dieses Design kann der Handgriff durch das Aufspannen eine Haltegraft auf dem Endoskophandgriff besitzen. Des Weiteren kann durch dieses Design auch auf unterschiedliche Formen von Endoskophandgriffen eingegangen werden. Insbesondere ermöglicht eine Aussparung in der Mitte (einem mittleren Bereich) des Handgriffs eine Positionierung direkt am Eingang eines internen Arbeitskanals des Endoskops (die Aussparung kann um den Eingang gelegt sein). Hierdurch ist der Handgriff in der Höhe (bzw. Axialerstreckung) positioniert (in proximal-distaler Richtung lagefestgelegt). Des Weiteren kann ein Befestigungsband (Verschlussband) benutzt werden/bereitgestellt sein, um den Handgriff in Gänze an dem Endoskop/Endoskophandgriff zu befestigen.

Des Weiteren hat der Handgriff bevorzugt sechs (in Längs- und/oder Umfangsrichtung verteilte) Rastposition, an die ein Konnektor (erste Montagekupplung) angebracht werden kann. Die unterschiedlichen Positionen sollen zum einen die unterschiedlichen Endoskoplängen ausgleichen (d.h., je nach Länge des zu adaptierenden Endoskops wird eine unterschiedliche Rastposition relativ zur Axialerstreckung des Handgriffs gewählt) und zum anderen dem Anwender die Möglichkeit geben, eigenen Präferenzen nachgehen zu können.

Der Konnektor ist (bildet) ein(en) Eingang für einen externen Schlauch (Arbeitskanal) und ist zweckmäßiger Weise mit einem LuerLock (Luer-Adapter) ausgebildet. Auf diesem LuerLock kann z.B. ein (Pentax-) Biopsieventil Platz finden. Die Befestigung des Konnektors am Handgriff wird bevorzugt durch Schnapphaken (Eingriffselementen der ersten Montagekupplung bzw. der Klemm-Branchenabschnitte) ermöglicht. Die Befestigung soll intuitiv und einfach gestaltet sein. Ferner kann die Befestigung / die Verbindung des Handgriffs und des Konnektors über (vorzugsweise gerippte) Flügel (Betätigungs-Branchenabschnitte) geöffnet werden, um den Konnektor umpositionieren zu können.

Zusammenfassend kann der Konnektor ein Luerlock, optional ein Biopsieventil, das bevorzugt auf den Luer-Lock aufsteckbar ist, eine Befestigungsmöglichkeit für ein BARS-System (d.h., einen Gewebeclip-Applikations-Adapter) und/oder einen zusätzlichen Arbeitskanal (AWC-System, "Additional Working Channel") aufweisen. Die Befestigung kann auf BARS/AWC als Teil mit integriert sein (d.h. ein integrales System bilden). Darüber hinaus kann der Konnektor Betätigungs-Klemmbranchen (d.h. die Flügel bzw. Betätigungs-Branchenabschnitte) haben.

### Figurenbeschreibung

Nachfolgend wird die vorliegende Offenbarung anhand bevorzugter Ausführungsformen beschrieben. Diese sind jedoch nur veranschaulichender Natur und sollen den Schutzumfang der vorliegenden Erfindung nicht einschränken. Ferner werden bei der Beschreibung der verschiedenen Ausführungsformen für gleiche Bauteile dieselben Bezugszeichen verwendet, um redundante Beschreibungen derselben zu vermeiden.
Fig. 1 zeigt ein Endsoskop mit einer daran fixierten adaptierbaren Arbeitskanalvorrichtung gemäß einem ersten Aspekt bzw. einer bevorzugten Ausführungsform.
Fig. 2 zeigt eine Draufsicht auf eine Griff-Montagemanschette der adaptierbaren Arbeitskanalvorrichtung nach der bevorzugten Ausführungsform.
Fig. 3 zeigt eine perspektivische Ansicht eines Konnektors der adaptierbaren Arbeitskanalvorrichtung.
Fig. 4 zeigt eine einstellbare Konfiguration der adaptierbaren Arbeitskanalvorrichtung nach der ersten Ausführungsform.
Fig. 5 zeigt ein Endoskop mit einem daran angebrachten Gewebeclip-Applikationsadapter nach einem zweiten Aspekt bzw. einer weiteren Ausführungsform.

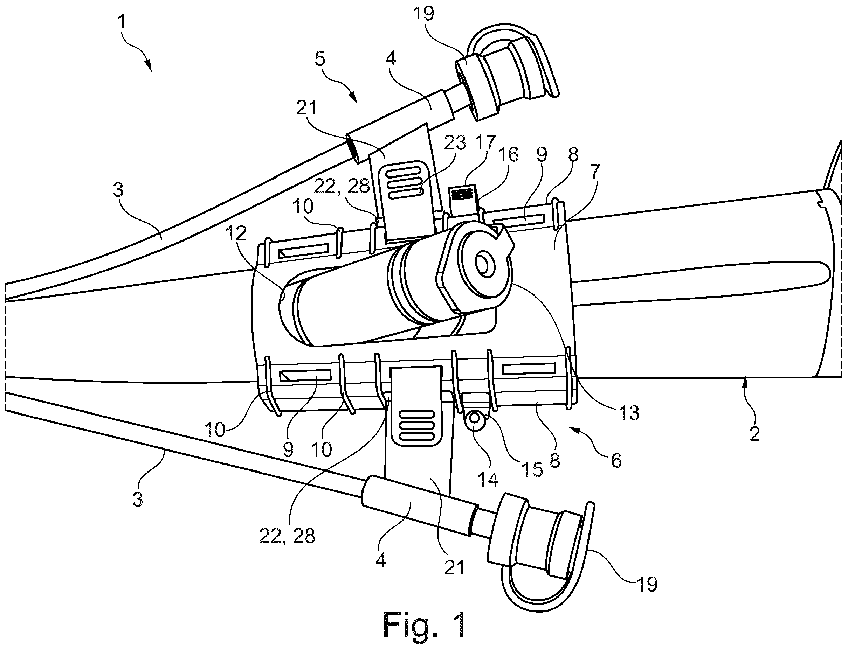

Fig. 1 zeigt eine adaptierbare Arbeitskanalvorrichtung 1, die ein Aus- oder Nachrüsten eines medizinischen Endoskops 2 mit zumindest einem externen Arbeitskanal 3 ermöglicht. In dieser Ausführungsform sind beispielhaft zwei (gleiche) Arbeitskanäle 3 bereitgestellt. Die Arbeitskanäle 3 sind jeweils separat von dem Endoskop 2 ausgebildet. Jeder externe Arbeitskanal 3 hat einen proximalen (d.h. einem Anwender zugewandt bzw. einem Patienten abgewandt) Endabschnitt. Dieser proximale Endabschnitt ist an eine Anschlusshülse 4 einer ersten Montagekupplung 5 angeschlossen. Die erste Montagekupplung 5 ist dazu ausgebildet, mit einer zweiten Montagekupplung 6 in lösbaren Halteeingriff zu kommen, welche am Endoskop 2 angebracht ist und nachfolgend genauer beschrieben wird.

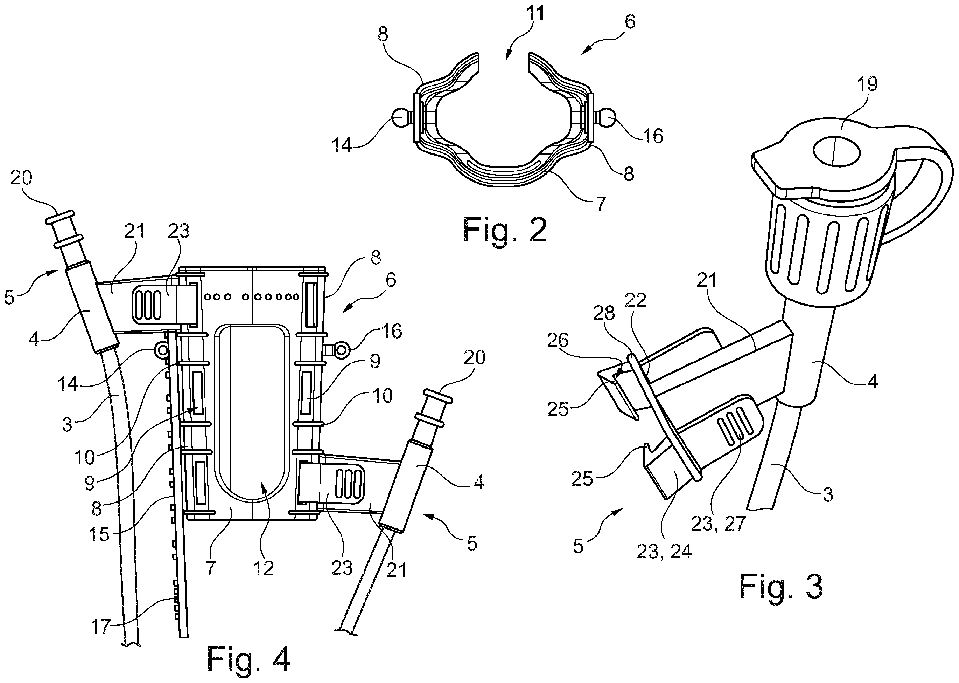

Die zweite Montagekupplung 6 ist an einer radialen Außenseite einer Griff-Montagemanschette 7 ausgebildet. Die Griff-Montagemanschette 7 ist im Wesentlichen hülsen- oder spangenartig ausgebildet und umgreift einen Teil des Endoskops 2, insbesondere, wie in Fig. 1 dargestellt, des Endoskopgriffs. Die Griff-Montagemanschette 7 bildet eine nach radial außen vorstehende eine Montageschiene 8 aus, in Längsrichtung der Griff-Montagemanschette 7 verläuft. In dieser Ausführungsform sind zwei (gleiche) einander gegenüberliegende Montageschienen 8 bereitgestellt. In Fig. 2 ist eine Draufsicht aus proximaler Richtung auf die Griff-Montagemanschette 7 dargestellt, in welcher gut erkennbar ist, dass die Montageschienen 8 in Form von Ausbuchtungen einer Umfangswand der Griff-Montageschiene 8 ausgebildet sind.

Die Montageschienen 8 weisen an ihren Flanken bzw. an ihren Seiten, welche in eine Umfangsrichtung der Griff-Adaptermanschette 7 ausgerichtet sind, Eingriffsöffnungen 9 auf. Die Eingriffsöffnungen 9 sind zum Eingriff mit der ersten Montagekupplung 5 konfiguriert/dimensioniert. Insbesondere erstrecken sich die Eingriffsöffnungen 9 durchgehend durch die gesamte Montageschiene 8, um sich an beiden Flanken der Montageschiene 8 zu öffnen. Die Montageschienen 8 bilden ferner Stützkanten 10 aus, welche in eine Richtung quer, insbesondere orthogonal, zur Längsrichtung der Griff-Montagemanschette 7 verlaufen. Die Stützkanten 10 sind derart angeordnet, dass sich an beiden Seiten jeder Eingriffsöffnungen 9, in der Längsrichtung betrachtet, jeweils eine der Stützkanten 10 befindet.

Die Griff-Montagemanschette 7 weist einen in Fig. 2 dargestellten Längsspalt 11 auf, welcher die Griff-Montagemanschette 7 entlang ihrer Längsrichtung an einer Umfangsposition vollständig durchbricht / spaltet. Somit kann die Griff-Montagemanschette 7 geweitet werden, um durch den Längsspalt 11 den Endoskopgriff aufzunehmen und darauf aufgeschnappt zu werden. Die Griff-Montagemanschette 7 weist, insbesondere an einer dem Längsspalt 11 gegenüberliegenden Seite, eine Aufnahmeöffnung 12 auf, die zur Aufnahme eines Radialfortsatzes 13 des Endoskops ausgebildet ist.

Ferner ist in einem Bereich zwischen zwei Stützkanten 10, an welchem bevorzugt keine der Eingriffsöffnungen 9 bereitgestellt ist, an einer radialen Außenseite einer der Montageschienen 8 eine Bandhalterung 14 zum Halten eines Verschlussbands 15 ausgebildet. Vorzugsweise ist das Verschlussband 15 an der Bandhalterung 14 translatorisch fest, optional aber um eine Achse quer zur Längsrichtung der Griff-Montagemanschette 7 drehbar angeordnet. Auf einer gleichen Höhe bezüglich der Längsrichtung ist an der gegenüberliegenden Montageschiene 8 ein Bandbefestigungsabschnitt 16 ausgebildet. Der Bandbefestigungsabschnitt 16 ist derart ausgebildet, dass daran ein Verschlussabschnitt 17 des Verschlussbands 15 befestigbar ist, wenn es den Längsspalt 11 überspannend um die Griff-Montagemanschette 7 geschlungen wird. In Fig. 1 ist das Verschlussband 15 in einer solchen den Längsspalt überspannenden, befestigten Stellung dargestellt, wobei lediglich Enden des Verschlussbands 15 zu sehen sind, welche entsprechend an der Bandhalterung 14 und dem Bandbefestigungsabschnitt 16 gehalten sind und hinter dem Endoskop hervorlugen. In Fig. 4 ist das Verschlussband 15 in einer herabhängenden Stellung dargestellt, in welcher es nicht am Bandbefestigungsabschnitt befestigt ist. Wie insbesondere aus Fig. 2 ersichtlich wird, können der Bandbefestigungsabschnitt 13 und/oder die Bandhalterung 14 Form von Knöpfen bereitgestellt sein.

Ein Aufbau der ersten Montagekupplung 5 ist insbesondere in Fig. 1 und Fig. 3 gut erkennbar. Genauer weist die erste Montagekupplung die Anschlusshülse 4 auf, an deren distalen Ende der externe Arbeitskanal 3 angeschlossen ist. Am proximalen Ende der Anschlusshülse 4 ist nach der dargestellten Konfiguration ein Biopsieventil 19 bereitgestellt. Alternativ oder zusätzlich kann, wie in Fig. 4 dargestellt, ein Luer-Adapter 20 ausgebildet sein.

Seitlich an der Anschlusshülse 4 erstreckt sich ein balkenartiger Abstandhalter 21 nach radial außen sowie vorzugsweise in Richtung distal, um eine schräge Anordnung des Arbeitskanals 3 und der Griff-Montagemanschette 7 bereitzustellen, wenn die erste und die zweite Montagekupplung 5, 6 miteinander verbunden sind. An einem von der Anschlusshülse 4 abgewandten Ende des Abstandhalters 21 ist ein Verbindungssteg 22 angeordnet, welcher sich in beide Richtungen quer zu dem Abstandhalter 21 erstreckt. D.h., der Abstandhalter 21 und der Verbindungssteg 22 sind T-förmig ausgebildet. An äußeren Enden des Verbindungsstegs 22 ist jeweils eine Klemmbranche 23 ausgebildet, welche sich zumindest abschnittsweise im Wesentlichen quer zu dem Verbindungssteg 22 bzw. im Wesentlichen parallel zu dem Abstandhalter 21 erstreckt. Der Verbindungssteg 22 stellt ein Wippgelenk bereit, um welches die Klemmbranchen 23 zueinander elastische winkelverstellbar sind.

Die Klemmbranchen 23 stehen in eine der Anschlusshülse 4 abgewandten Richtung (d.h. nach radial außen bezüglich der Anschlusshülse 4) über das Wippgelenk bzw. den Verbindungssteg 22 vor, um Klemm-Branchenabschnitte 24 zu bilden. Die Klemm-Branchenabschnitte 24 sind leicht nach außen, d.h. voneinander weg, abgewinkelt. Ferner bilden die Klemm-Branchenabschnitte 24 jeweils einen hakenförmigen Fortsatz 25 aus, welcher dem entsprechend anderen Klemm-Branchenabschnitt 24 zugewandt ist und zum Eingriff in die Eingriffsöffnungen 9 der zweiten Montagevorrichtung 6 ausgebildet. Die Klemmbranchen 23 sind in einer Ruhelage in einer geschlossenen Stellung, in welcher die Klemm-Branchenabschnitte 24, vorzugsweise maximal, aufeinander zubewegt sind. Eine der Anschlusshülse 4 zugewandte Kontaktfläche 26 des jeweiligen Fortsatzes 25 kommt an einer entsprechenden Kontaktfläche einer der Eingriffsöffnungen 9 der zweiten Montagevorrichtung 6 zur Anlage, wenn die erste und zweite Montagevorrichtung 5, 6 miteinander gekoppelt werden. Die Kontaktfläche 26 der Fortsätze 25 ist im Wesentlichen senkrecht zu einer Erstreckungsrichtung des Abstandhalters 21 ausgebildet, um möglichst hohe Kräfte übertragen zu können.

Die Klemmbranchen 23 stehen in eine der Anschlusshülse 4 zugewandten Richtung (d.h. nach radial innen bezüglich der Anschlusshülse 4) über das Wippgelenk bzw. den Verbindungssteg 22 vor, um Betätigungs-Branchenabschnitte 27 zu bilden. Die Betätigungs-Branchenabschnitte 27 und die Klemm-Branchenabschnitte 24 sind steif miteinander verbunden. Werden die Betätigungs-Branchenabschnitte 27, vorzugsweise einhändig, durch einen Anwender gedrückt / aufeinander zubewegt, werden die Klemmbranchen 23 um den Verbindungssteg 22 bzw. das Wippgelenk verkippt und die Klemm-Branchenabschnitte 24 bewegen sich voneinander weg, um einen Klemmeingriff derselben mit den Eingriffsöffnungen 9 lösen zu können. Die Betätigungs-Branchenabschnitte 27 weisen eine außenseitige Riffelung auf, welche ein sicheres, abrutschfreies Betätigen der Betätigungs-Branchenabschnitte 27 ermöglicht.

Der Verbindungssteg 22 hat Seitenränder 28, welche seitlich, d.h. in proximaler und in distaler Richtung, über die Klemmbranchen 22 vorstehen. Eine Breite des Verbindungsstegs 22 in dieser Richtung entspricht im Wesentlichen einem Abstand zwischen den Stützkanten 10 der zweiten Montagekupplung 6. Im verbundenen Zustand der ersten und zweiten Montagekupplungen 5, 6 sind somit die Stützkanten 10 der zweiten Montagekupplung 6 und die Seitenränder 28 des Verbindungsstegs 22 unmittelbar benachbart und insbesondere in Anlage miteinander, wenn, wie in Fig. 1 erkennbar, eine Belastung des Arbeitskanals 3 in Längsrichtung auftritt. Seitliche, in proximal-distaler Richtung ausgerichtete Ränder der Klemm-Branchenabschnitte 24 kommen dabei nicht zur Anlage an einer Seite der jeweiligen Eingriffsöffnung 9, was die Kraftübertragung zwischen der ersten und zweiten Montagemanschette 5, 6 verbessert.

Fig. 1 und Fig. 4 zeigen unterschiedliche Konfigurationen der im Wesentlichen gleich ausgebildeten adaptiven Arbeitskanalvorrichtung. In Fig. 1 sind zwei Arbeitskanäle 3 auf einer gleichen Höhe zueinander mit der zweiten Montagekupplung 6 verbunden. In Fig. 4 sind die Arbeitskanäle 3 an zueinander in Längsrichtung versetzten Positionen eines durch die Eingriffsöffnungen 9 definierten Rasters angeordnet.

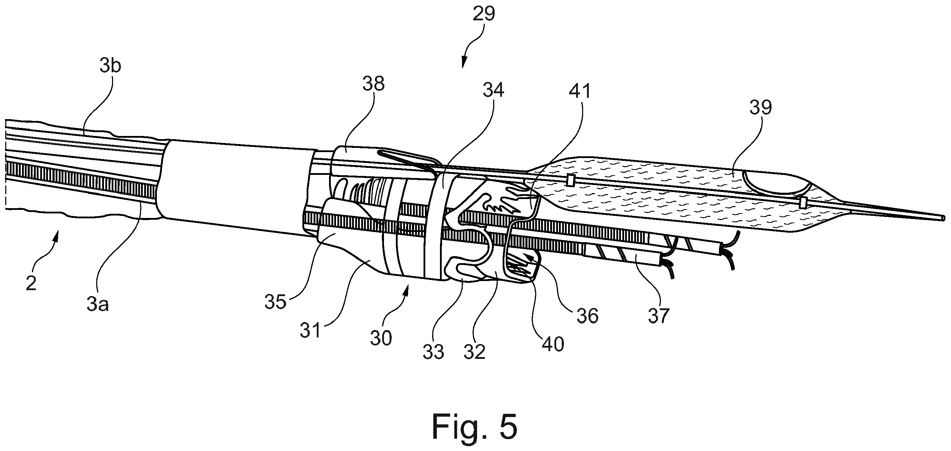

Fig. 5 zeigt ein Endsokop 2, genauer, einen Endoskopschaft, an dessen distalem Endoskopkopf ein Gewebeclip-Applikationsadapter 29 nachgerüstet ist, um das Endsokop 2 um eine Funktionalität, Patientengewebe zu klippen, zu erweitern. Der Gewebeclip-Applikationsadapter 29 ist in Form eines kappenartigen Aufsatzes bereitgestellt und weist einen hülsenförmigen Grundkörper 30 auf. Der Grundkörper 30 hat einen proximalen Endoskophalteabschnitt 31, welcher zur Befestigung an dem Endoskopkopf ausgebildet ist, und einen Gewebeclip-Halteabschnitt 32, auf welchem ein Gewebeclip 33 getragen wird. Unmittelbar proximal zu dem Gewebeclip 33 bzw. dem Gewebeclip-Halteabschnitt 32 ist eine Schiebehülse 34 längsverschiebbar auf dem Grundkörper 30 gelagert. Wir die Schiebehülse 34 in Richtung distal verschoben, kontaktiert sie den Gewebeclip 33 und schiebt diesen in distaler Richtung von dem Gewebeclip-Applikationsadapter 29 herunter.

Ein erster Adapter-eigener Arbeitskanal-Anschluss 35 hat einen ersten Innenkanal, der zur Kopplung mit einem ersten externen Arbeitskanal 3a ausgebildet ist. Der erste Innenkanal erstreckt sich aus Richtung proximal kommend von einer Außenseite des Grundkörpers 30 in Richtung distal und radial innen durchgängig durch eine Wandung des Grundkörpers 30 und mündet in dessen Innenraum 36. D.h., ein proximales erstes Kanalende öffnet sich an einer Außenseite des Grundkörpers 30 und ein distales erstes Kanalende öffnet an einer Innenseite des Grundkörpers 30. Bevorzugt ist ein Greifinstrument 37 bereitgestellt, welches in den ersten Arbeitskanal 3a eingeschoben ist oder werden kann, um durch den ersten Innenkanal in den Innenraum 36 und ggf. distal aus dem Grundkörper 30 hinaus vorgeschoben zu werden.

Ein zweiter Adapter-eigener Arbeitskanal-Anschluss 38 hat einen zweiten Innenkanal, der zur Kopplung mit einem zweiten externen Arbeitskanal 3b ausgebildet ist. Der zweite Innenkanal erstreckt sich aus Richtung proximal kommend entlang einer Außenseite des Grundkörpers 30 in Richtung distal. Er mündet außenseitig an dem Grundkörper 30 an einer Position, welche proximal zu dem Gewebeclip-Halteabschnitt 32 angeordnet ist. D.d., sowohl ein proximales zweites Kanalende als auch ein distales zweites Kanalende öffnen sich an einer Außenseite des Grundkörpers 30. Bevorzugt ist ein Spreizinstrument 39, wie z.B. ein Ballonkatheter, bereitgestellt, welches in den zweiten Arbeitskanal 3a eingeschoben ist oder werden kann, um durch den zweiten Arbeitskanalanschluss 35 außerhalb des Grundkörpers 30 und seitlich neben dem Gewebeclip-Halteabschnitt 32 bereitstellbar zu sein.

Der Grundkörper 30 hat ferner eine distale Stirnkante, welche zwei einander diametral gegenüberliegende Rücksprünge 40 aufweist, die sich in proximaler Richtung erstrecken und ein Falten von Patientengewebe ermöglichen, das in den Innenraum 36 gezogen wird. Zwischen den Rücksprüngen 40 sind Vorsprünge bereitgestellt, deren Außenflächen als Spreizstützflächen 41 zum Offenhalten bzw. Spreizen des zusammenklappbaren Gewebeclips 33 ausgebildet sind. Der Gewebeclip 33 hat Krallenabschnitte und ist dazu ausgebildet, dass diese Krallenabschnitte aufeinander zu klappen, wenn der Gewebeclip 33 von dem Gewebeclip-Halteabschnitt 32 abgeschoben wird. Die Krallenabschnitte sind somit auf den Spreizstützflächen 41 gestützt. Eine der Spreizstützflächen 41 ist insbesondere an einer Umgangsposition angeordnet, an welcher sich das distale zweite Kanalende öffnet.

Die ersten und zweiten externen Arbeitskanäle 3a, 3b sind bevorzugt externe Arbeitskanäle 3 einer adaptierbaren Arbeitskanalvorrichtung 1 gemäß Fig. 1. Somit bilden die adaptierbare Arbeitskanalvorrichtung 1 und der Gewebeclip-Applikationsadapter 29 nach Fig. 4 ein chirurgisches Ausrüstsystem, welches insbesondere ferner das Greifinstrument 37 und das Spreizinstrument 39 aufweist.

### Referenzzeichenliste

- 1: Adaptierbare Arbeitskanalvorrichtung
- 2: Endoskop
- 3: Arbeitskanal
- 3a: Erster externer Arbeitskanal des chirurgischen Ausrüstsystems
- 3b: Zweiter externer Arbeitskanal des chirurgischen Ausrüstsystems
- 4: Anschlusshülse
- 5: Erste Montagekupplung
- 6: Zweite Montagekupplung
- 7: Griff-Montagemanschette
- 8: Montageschiene
- 9: Eingriffsöffnung / Eingriffselement der zweiten Montagekupplung
- 10: Stützkanten
- 11: Längsspalt
- 12: Hinterschnitt / Aufnahmeöffnung
- 13: Radialfortsatz
- 14: Bandhalterung
- 15: Verschlussband
- 16: Bandbefestigungsabschnitt
- 17: Verschlussabschnitt
- 19: Biopsieventil
- 20: Luer-Adapter
- 21: Abstandhalter / zentraler Balken
- 22: Verbindungssteg
- 23: Klemmbranchen
- 24: Klemm-Branchenabschnitt
- 25: Hakenartiger Fortsatz / Eingriffselement der ersten Montagekupplung
- 26: Kontaktfläche
- 27: Betätigungs-Branchenabschnitt
- 28: Vorstehender Seitenrand des Verbindungsstegs
- 29: Gewebeclip-Applikationsadapter
- 30: Hülsenartiger Grundkörper
- 31: Proximaler Endoskop-Halteabschnitt
- 32: Gewebeclip-Halteabschnitt
- 33: Gewebeclip
- 34: Schiebehülse
- 35: Erster Adapter-eigener Arbeitskanal-Anschluss
- 36: Innenraum
- 37: Greifinstrument
- 38: Zweiter Adapter-eigener Arbeitskanal-Anschluss
- 39: Spreizinstrument / Ballonkatheter
- 40: Rücksprünge der distalen Stirnkante
- 41: Spreizstützflächen

## Patentansprüche

1. Adaptierbare Arbeitskanalvorrichtung (1) zur adaptiven Montage an einem Endoskop (2) mit
zumindest einem externen Arbeitskanal (3), der an seinem proximalen Endabschnitt an eine erste Montagekupplung (5) angeschlossen ist,
einer separat zum Arbeitskanal (3) ausgebildeten Griff-Montagemanschette (7), welche dafür vorgesehen ist, einen Endoskopschaft oder einen Endoskopgriff manschettenartig zu umgreifen und welche an ihrer radial äußeren Umfangsseite zumindest eine zweite Montagekupplung (6) hat, die ausgebildet ist, um mit der ersten Montagekupplung (5) in einen lösbaren Montageeingriff zu kommen,
wobei die erste Montagekupplung (5) zwei in einen Klemmeingriff federvorgespannte Klemmbranchen (23) hat, die wäscheklammerartig aneinanderscharniert sind und dabei jeweils einen Klemm-Branchenabschnitt (24) und einen manuellen Betätigungs-Branchenabschnitt (27) bilden, und
die zweite Montagekupplung (6) eine Montageschiene (8) hat, die sich in Längsrichtung der Griff-Montagemanschette (7) erstreckt und zumindest einen Hinterschnitt (9) aufweist oder ausbildet, der derart dimensioniert ist, dass er formschlüssig von den Klemmbranchen (23) der ersten Montagekupplung (5) umgreifbar ist.

2. Adaptierbare Arbeitskanalvorrichtung (1) nach Anspruch 1, wobei die Klemmbranchen (23) jeweils an einer Position zwischen dem Klemm-Branchenabschnitt (24) und dem Betätigungs-Branchenabschnitt (27) der entsprechenden Klemmbranche (23) über einen Verbindungssteg (22) elastisch miteinander verbunden sind, der schräg oder senkrecht zu den Klemmbranchen (23) verläuft.

3. Adaptierbare Arbeitskanalvorrichtung (1) nach Anspruch 2, wobei
der Verbindungssteg (22) derart breiter als die Klemmbranchen (23) ist, dass er seitlich über die Klemmbranchen (23) vorsteht, und
die Griff-Montagemanschette (7) zumindest zwei quer zu ihrer Längsrichtung verlaufende und in der Längsrichtung beabstandete Stützkanten (10) ausbildet, deren Abstand zueinander im Wesentlichen einer Breite des Verbindungsstegs (22) entspricht.

4. Adaptierbare Arbeitskanalvorrichtung (1) nach einem der vorstehenden Ansprüche, wobei die erste Montagekupplung eine Anschlusshülse (4) ausbildet, an deren distalem Ende der Arbeitskanal (3) angeschlossen ist und and deren proximalem Ende ein Luer-Adapter (20) oder ein integriertes Biopsieventil (19) ausgebildet ist.

5. Adaptierbare Arbeitskanalvorrichtung (1) nach einem der vorstehenden Ansprüche, wobei die Montageschiene (8) der Griff-Montagemanschette (7) mehrere Hinterschnitte (9) ausbildet, die in der Längsrichtung zueinander versetzt angeordnet sind.

6. Adaptierbare Arbeitskanalvorrichtung (1) nach einem der vorstehenden Ansprüche, wobei die Griff-Montagemanschette (7) mehrere Montageschienen (8) ausbildet, die in einer Umfangsrichtung der Griff-Montagemanschette (7) zueinander versetzt, vorzugsweise einander diametral gegenüberliegend, angeordnet sind.

7. Adaptierbare Arbeitskanalvorrichtung (1) nach einem der vorstehenden Ansprüche, wobei die Griff-Montagemanschette (7) eine Aufnahmeöffnung () aufweist, welche dazu ausgebildet ist, einen Radialfortsatz (13) des Endoskopschafts oder Endoskopgriffs aufzunehmen.

8. Adaptierbare Arbeitskanalvorrichtung (1) nach einem der vorstehenden Ansprüche, wobei die Griff-Montagemanschette (7) einen durchgehenden Längsspalt (11) aufweist und zumindest teilweise elastisch ausgebildet ist, derart, dass die Griff-Montagemanschette (7) spangenartig auf den Endoskopgriff oder Endoskopschaft aufschnappbar ist.

9. Adaptierbare Arbeitskanalvorrichtung (1) nach Anspruch 8, wobei die Griff-Montagemanschette (7)
eine Bandhalterung (14), an welcher ein erstes Ende eines Verschlussbands (15) angebracht oder anbringbar ist, und
einen Bandbefestigungsabschnitt (16) aufweist, welcher dazu ausgebildet ist, einen Verschlussabschnitt (17) des Verschlussbands (15) in einer Position zu halten, in welcher das Verschlussband (15) derart um die Griff-Montagemanschette gelegt ist, dass es sich über den Längsspalt (11) erstreckt.

10. Gewebeclip-Applikationsadapter (29), der nach Art eines Kappenaufsatzes für ein medizinisches Endoskop (2) ausgebildet ist, mit
einem hülsenartigen Grundkörper (30), welcher einen proximalen Endoskop-Halteabschnitt (31) aufweist, der dazu ausgebildet ist, an einem distalen Kopf des medizinischen Endoskops (2) angebracht zu werden, und welcher ferner einen Gewebeclip-Halteabschnitt (32) aufweist, der dazu ausgebildet ist, auf seiner radial äußeren Umfangsfläche einen Gewebeclip (33) derart zu tragen, dass dieser in distaler Richtung von dem Gewebeclip-Halteabschnitt (32) entfernbar ist, sowie mit
einem ersten Adapter-eigenen Arbeitskanal-Anschluss (35) mit einem ersten Innenkanal, der sich derart durch den Grundkörper (30) erstreckt, dass ein proximales erstes Kanalende sich an einer Außenseite des Grundkörpers (30) öffnet und ein distales erstes Kanalende in einem Innenraum innerhalb des Grundkörpers (30) mündet,
und einem zweiten Adapter-eigenen Arbeitskanal-Anschluss (38), mit einem zweiten Innenkanal, der sich derart entlang des Grundkörpers (30) erstreckt, dass ein proximales zweites Kanalende und ein distales zweites Kanalende sich an einer Außenseite des Grundkörpers (30) öffnen und dass das distale zweite Kanalende proximal zu dem Gewebeclip-Halteabschnitt (32) angeordnet ist.

11. Gewebeclip-Applikationsadapter (29) nach Anspruch 10, wobei unmittelbar proximal zu dem Gewebeclip-Halteabschnitt (32) eine Schiebehülse (34) in Längsrichtung verschiebbar auf dem Grundkörper gelagert ist, um den Gewebeclip (33) in distaler Richtung von dem Gewebeclip-Halteabschnitt (32) herunterzuschieben.

12. Gewebeclip-Applikationsadapter (29) nach einem der Ansprüche 10 und 11, wobei eine distale Stirnkante des Grundkörpers (30) zwei einander diametral gegenüberliegende Rücksprünge (40) aufweist, welche sich in proximaler Richtung erstrecken.

13. Gewebeclip-Applikationsadapter (29) nach Anspruch 12, wobei die Rücksprünge (40) jeweils in einander entgegengesetzten Umfangsrichtungen des Grundkörpers (30) versetzt zum zweiten Adapter-eigenen Arbeitskanal-Anschluss (38) angeordnet sind, vorzugsweise um im 70° bis 110°, weiter vorzugsweise um 90° dazu versetzt sind.

14. Gewebeclip-Applikationsadapter (29) nach einem der Ansprüche 10 bis 13, wobei der Gewebeclip-Halteabschnitt (32) zwei einander diametral gegenüberliegende Spreizstützflächen (41) ausbildet, welche dazu konfiguriert sind, den Gewebeclip (33) in einer vorbestimmten Lage aufgespreizt zu halten, wobei eine der Spreizstützflächen (41) auf einer gleichen Umfangsposition des Grundkörpers (30) wie der zweite Adapter-eigene Arbeitskanal-Anschluss (38) angeordnet ist.

15. Chirurgisches Ausrüstsystem für ein Endoskop (2) **gekennzeichnet durch**
eine Adaptierbare Arbeitskanalvorrichtung (1) nach Anspruch 1,
einem Gewebeclip-Applikationsadapter (29) nach Anspruch 2, dessen erster Adapter-eigener Arbeitskanal-Anschluss (35) mit einem distalen Endabschnitt des externen Arbeitskanals (3) der adaptierbaren Arbeitskanalvorrichtung (1) als einem ersten externen Arbeitskanal (3a) gekoppelt ist,
einem Greifinstrument (37), welches durch den ersten externen Arbeitskanal (3a) in Richtung distal eingeschoben ist oder dazu ausgebildet ist, in den ersten externen Arbeitskanal (3a) eingeschoben zu werden, um durch den Innenraum (36) des Grundkörpers (30) des Gewebeclip-Applikationsadapters (29) vorschiebbar zu sein
einem zweiten externen Arbeitskanal (3b), welcher an seinem proximalen Endabschnitt eine dritte Montagekupplung zur Montage an der Griff-Montagemanschette (7) der adaptierbaren Arbeitskanalvorrichtung (1) aufweist und mit seinem distalen Endabschnitt mit dem zweiten Adapter-eigenen Arbeitskanal-Anschluss (38) des Gewebeclip-Applikationsadapters (29) gekoppelt ist, und
einem Spreizinstrument (39), welches in den zweiten externen Arbeitskanal (3b) in Richtung distal eingeschoben ist oder dazu ausgebildet ist, in den zweiten externen Arbeitskanal (3b) eingeschoben zu werden, um radial außerhalb des Grundkörpers (30) des Gewebeapplikations-Adapters (29) bereitstellbar zu sein.

## Claims

1. An adaptable working channel apparatus (1) for adaptive mounting to an endoscope (2) with
at least one external working channel (3) connected at its proximal end portion to a first mounting coupling (5),
a handle-mounting sleeve (7) formed separately from the working channel (3) and provided to encompass an endoscope shaft or an endoscope handle like a sleeve and which has on its radially outer circumferential side at least a second mounting coupling (6) which is configured to come into releasable mounting engagement with the first mounting coupling (5),
wherein
the first mounting coupling (5) has two clamping branches (23) spring-loaded into a clamping engagement, hinged together in a clothespin-like manner, to form a respective clamping branch portion (24) and a manual actuation branch portion (27), and
the second mounting coupling (6) has a mounting rail (8) extending in a longitudinal direction of the handle-mounting sleeve (7) and has or forms at least one undercut (9) which is dimensioned such that it can be form-fittingly embraced by the clamping branches (23) of the first mounting coupling (5).

2. The adaptable working channel apparatus (1) according to claim 1, wherein the clamping branches (23) are respectively elastically connected to each other at a position between the clamping-branch portion (24) and the actuation branch portion (27) of the corresponding clamping branch (23) via a connecting web (22) that extends obliquely or perpendicularly to the clamping branches (23).

3. The adaptable working channel apparatus (1) according to claim 2, wherein
the connecting web (22) is wider than the clamping branches (23) such that it projects laterally beyond the clamping branches (23), and
the handle-mounting sleeve (7) forms at least two support edges (10) extending transversely to its longitudinal direction and spaced apart in the longitudinal direction, the distance between them corresponding substantially to a width of the connecting web (22).

4. The adaptable working channel apparatus (1) according to one of the preceding claims, wherein the first mounting coupling forms a connection bush (4), to the distal end of which the working channel (3) is connected and to the proximal end of which a Luer adapter (20) or an integrated biopsy valve (19) is formed.

5. The adaptable working channel apparatus (1) according to one of the preceding claims, wherein the mounting rail (8) of the handle-mounting sleeve (7) forms a plurality of undercuts (9) that are arranged offset to each other in the longitudinal direction.

6. The adaptable working channel apparatus (1) according to one of the preceding claims, wherein the handle-mounting sleeve (7) forms a plurality of mounting rails (8) that are arranged offset to each other in a circumferential direction of the handle-mounting sleeve (7), preferably diametrically opposite each other.

7. The adaptable working channel apparatus (1) according to one of the preceding claims, wherein the handle-mounting sleeve (7) has a receiving opening () configured to receive a radial protrusion (13) of the endoscope shaft or of the endoscope handle.

8. The adaptable working channel apparatus (1) according to one of the preceding claims, wherein the handle-mounting sleeve (7) has a continuous longitudinal gap (11) and is at least partially elastic, such that the handle-mounting sleeve (7) can be snapped onto the endoscope handle or endoscope shaft in a clasp-like manner.

9. The adaptable working channel apparatus (1) according to claim 8, wherein the handle-mounting sleeve (7) comprises
a strap holder (14) to which a first end of a closure strap (15) is attached or attachable, and
a strap fixing portion (16) configured to hold a closure portion (17) of the closure strap (15) in a position in which the closure strap (15) is wrapped around the handle-mounting sleeve such that it extends across the longitudinal gap (11).

10. A tissue-clip application adapter (29) configured in the manner of a cap attachment for a medical endoscope (2), with
a bush-like base body (30) having a proximal endoscope holding portion (31) configured to be attached to a distal head of the medical endoscope (2), and furthermore having a tissue-clip holding portion (32) configured to support a tissue clip (33) on its radially outer circumferential surface such that tissue clip (33) is removable in the distal direction from the tissue-clip holding portion (32), and
a first adapter-integrated working-channel terminal (35) having a first inner channel extending through the base body (30) such that a proximal first channel end opens at an exterior of the base body (30) and a distal first channel end opens at an interior within the base body (30),
and a second adapter-integrated working-channel terminal (38) having a second inner channel extending along the base body (30) such that a proximal second channel end and a distal second channel end open at an outer side of the base body (30) and that the distal second channel end is disposed proximal to the tissue-clip holding portion (32).

11. The tissue-clip application adapter (29) according to claim 10, wherein a sliding bush (34) is mounted in a longitudinally slidable manner on the base body immediately proximal to the tissue-clip holding portion (32) for sliding the tissue clip (33) distally off the tissue-clip holding portion (32).

12. The tissue-clip application adapter (29) according to one of claims 10 and 11, wherein a distal front edge of the base body (30) has two diametrically opposite recesses (40) which extend in a proximal direction.

13. The tissue-clip application adapter (29) according to claim 12, wherein the recesses (40) are each arranged offset from the second adapter-integrated working-channel terminal (38) in opposite circumferential directions of the base body (30), preferably by 70° to 110°, more preferably by 90°.

14. The tissue-clip application adapter (29) according to one of claims 10 to 13, wherein the tissue-clip holding portion (32) forms two diametrically opposed spread support surfaces (41) configured to hold the tissue clip (33) spread open in a predetermined pose, wherein one of the spread support surfaces (41) is disposed at an identical circumferential position of the base body (30) as the second adapter-integrated working-channel terminal (38).

15. A surgical equipping system for an endoscope (2) **characterized by**
an adaptable working channel apparatus (1) according to claim 1,
a tissue-clip application adapter (29) according to claim 2, the first adapter-integrated working-channel terminal (35) of which is coupled to a distal end portion of the external working channel (3) of the adaptable working channel apparatus (1) as a first external working channel (3a),
a gripping instrument (37) inserted or configured to be inserted distally through the first external working channel (3a) so as to be advanceable through the interior (36) of the base body (30) of the tissue-clip application adapter (29),
a second external working channel (3b) having a third mounting coupling at its proximal end portion for mounting to the handle-mounting sleeve (7) of the adaptable working channel apparatus (1) and having its distal end portion coupled to the second adapter-integrated working-channel terminal (38) of the tissue-clip application adapter (29), and
a spreading instrument (39) which is inserted or configured to be inserted into the second external working channel (3b) in a distal direction to be providable radially outside the base body (30) of the tissue application adapter (29).

## Revendications

1. Dispositif à canal de travail (1) adaptable pour le montage adaptatif au niveau d'un endoscope (2) avec au moins un canal de travail externe (3) qui est raccordé au niveau de sa section terminale proximale à un premier couplage de montage (5),
une manchette de montage de poignée (7) réalisée séparément du canal de travail (3) qui est prévue afin d'entourer comme une manchette une tige d'endoscope ou une poignée d'endoscope et qui présente sur son côté périphérique radialement extérieur au moins un deuxième couplage de montage (6) qui est réalisé afin de venir en prise de montage amovible avec le premier couplage de montage (5),
dans lequel le premier couplage de montage (5) présente deux branches de serrage (23) précontraintes par ressort dans une prise de serrage, lesquelles sont articulées l'une à l'autre comme une pince à linge et forment respectivement une section de branche de serrage (24) et une section de branche d'actionnement (27) manuelle, et
le deuxième couplage de montage (6) présente un rail de montage (8) qui s'étend dans le sens longitudinal de la manchette de montage de poignée (7) et présente ou réalise au moins une contre-dépouille (9) qui est dimensionnée de telle manière qu'elle puisse être entourée par complémentarité de formes par les branches de serrage (23) du premier couplage de montage (5).

2. Dispositif à canal de travail (1) adaptable selon la revendication 1, dans lequel les branches de serrage (23) sont reliées élastiquement les unes aux autres respectivement au niveau d'une position entre la section de branche de serrage (24) et la section de branche d'actionnement (27) des branches de serrage (23) correspondantes par le biais d'une barrette de liaison (22) qui s'étend en biais ou perpendiculairement aux branches de serrage (23).

3. Dispositif à canal de travail (1) adaptable selon la revendication 2, dans lequel la barrette de liaison (22) est plus large que les branches de serrage (23) de telle manière qu'elle dépasse latéralement des branches de serrage (23), et
la manchette de montage de poignée (7) réalise au moins deux arêtes d'appui (10) espacées dans le sens longitudinal et s'étendant transversalement à son sens longitudinal, dont la distance l'une part rapport à l'autre correspond sensiblement à une largeur de la barrette de liaison (22).

4. Dispositif à canal de travail (1) adaptable selon l'une quelconque des revendications précédentes, dans lequel le premier couplage de montage réalise une douille de raccordement (4) à l'extrémité distale de laquelle le canal de travail (3) est raccordé et à l'extrémité proximale de laquelle un adaptateur Luer (20) ou une valve de biopsie (19) intégrée est réalisée.

5. Dispositif à canal de travail (1) adaptable selon l'une quelconque des revendications précédentes, dans lequel le rail de montage (8) de la manchette de montage de poignée (7) réalise plusieurs contre-dépouilles (9) qui sont agencées en déport les unes des autres dans le sens longitudinal.

6. Dispositif à canal de travail (1) adaptable selon l'une quelconque des revendications précédentes, dans lequel la manchette de montage de poignée (7) réalise plusieurs rails de montage (8) qui sont agencés en déport les uns des autres dans un sens périphérique de la manchette de montage de poignée (7), de préférence de manière diamétralement opposée les uns aux autres.

7. Dispositif à canal de travail (1) adaptable selon l'une quelconque des revendications précédentes, dans lequel la manchette de montage de poignée (7) présente une ouverture de réception () qui est réalisée afin de recevoir un prolongement radial (13) de la tige d'endoscope ou poignée d'endoscope.

8. Dispositif à canal de travail (1) adaptable selon l'une quelconque des revendications précédentes, dans lequel la manchette de montage de poignée (7) présente une fente longitudinale (11) continue et est réalisée au moins partiellement élastiquement de telle manière que la manchette de montage de poignée (7) puisse être encliquetée comme une griffe sur la poignée d'endoscope ou tige d'endoscope.

9. Dispositif à canal de travail (1) adaptable selon la revendication 8, dans lequel la manchette de montage de poignée (7) présente un support de bande (14) au niveau duquel une première extrémité d'une bande de fermeture (15) est ou peut être montée, et
une section de fixation de bande (16) qui est réalisée afin de maintenir une section de fermeture (17) de la bande de fermeture (15) dans une position dans laquelle la bande de fermeture (15) est placée autour de la manchette de montage de poignée de telle manière qu'elle s'étende au-delà de la fente longitudinale (11).

10. Adaptateur d'application de pince à tissu (29) qui est réalisé comme un embout de capuchon pour un endoscope (2) médical, avec
un corps de base (30) de type douille qui présente une section de retenue d'endoscope (31) proximale qui est réalisée afin d'être montée au niveau d'une tête distale de l'endoscope (2) médical, et qui présente de plus une section de retenue de pince à tissu (32) qui est réalisée afin de porter sur sa surface périphérique radialement extérieure une pince à tissu (33) de telle manière que celle-ci puisse être retirée de la section de retenue de pince à tissu (32) dans le sens distal, ainsi qu'avec
un premier raccord de canal de travail (35) propre à l'adaptateur avec un premier canal intérieur qui s'étend à travers le corps de base (30) de telle manière qu'une première extrémité de canal proximale s'ouvre au niveau d'un côté extérieur du corps de base (30) et une première extrémité de canal distale débouche dans un espace intérieur à l'intérieur du corps de base (30),
et un second raccord de canal de travail (38) propre à l'adaptateur avec un second canal intérieur qui s'étend le long du corps de base (30) de telle manière qu'une seconde extrémité de canal proximale et une seconde extrémité de canal distale s'ouvrent au niveau d'un côté extérieur du corps de base (30) et que la seconde extrémité de canal distale soit agencée de manière proximale à la section de retenue de pince à tissu (32).

11. Adaptateur d'application de pince à tissu (29) selon la revendication 10, dans lequel une douille coulissante (34) est logée de manière coulissante dans le sens longitudinal sur le corps de base de manière directement proximale à la section de retenue de pince à tissu (32) afin de pousser la pince à tissu (33) dans le sens distal de la section de retenue de pince à tissu (32).

12. Adaptateur d'application de pince à tissu (29) selon l'une quelconque des revendications 10 et 11, dans lequel une arête avant distale du corps de base (30) présente deux retraits (40) opposés diamétralement l'un à l'autre qui s'étendent dans le sens proximal.

13. Adaptateur d'application de pince à tissu (29) selon la revendication 12, dans lequel les retraits (40) sont agencés respectivement dans des sens périphériques opposés l'un à l'autre du corps de base (30) en déport du second raccord de canal de travail (38) propre à l'adaptateur, de préférence sont en déport de 70° à 110°, plus préférentiellement de 90°.

14. Adaptateur d'application de pince à tissu (29) selon l'une quelconque des revendications 10 à 13, dans lequel la section de retenue de pince à tissu (32) réalise deux surfaces d'appui d'écartement (41) opposées diamétralement l'une à l'autre qui sont configurées afin de maintenir écartée la pince à tissu (33) dans une position prédéterminée, dans lequel une des surfaces d'appui d'écartement (41) est agencée sur une position périphérique identique du corps de base (30) et du second raccord de canal de travail (38) propre à l'adaptateur.

15. Système d'équipement chirurgical pour un endoscope (2) **caractérisé par** un dispositif à canal de travail (1) adaptable selon la revendication 1,
un adaptateur d'application de pince à tissu (29) selon la revendication 2, dont le premier raccord de canal de travail (35) propre à l'adaptateur est couplé à une section terminale distale du canal de travail (3) externe du dispositif à canal de travail (1) adaptable comme un premier canal de travail (3a) externe,
un instrument de préhension (37) qui est enfoncé à travers le premier canal de travail (3a) externe en direction distale ou est réalisé afin d'être enfoncé dans le premier canal de travail (3a) externe afin de pouvoir être avancé à travers l'espace intérieur (36) du corps de base (30) de l'adaptateur d'application de pince à tissu (29)
un second canal de travail (3b) externe qui présente au niveau de sa section terminale proximale un troisième couplage de montage pour le montage au niveau de la manchette de montage de poignée (7) du dispositif à canal de travail (1) adaptable et est couplé avec sa section terminale distale au second raccord de canal de travail (38) propre à l'adaptateur de l'adaptateur d'application de pince à tissu (29), et
un instrument d'écartement (39) qui est enfoncé dans le second canal de travail (3b) externe en direction distale ou est réalisé afin d'être enfoncé dans le second canal de travail (3b) externe afin de pouvoir être mis à disposition radialement à l'extérieur du corps de base (30) de l'adaptateur d'application de pince à tissu (29).
